# EUROPEAN PATENT APPLICATION

(11) **EP 4 527 285 A1**
(43) Date of publication of application: **26.03.2025**
(21) Application number: 23807376.1
(22) Date of filing: 20.04.2023
(51) Int. Cl.: A61B 1/07, A61B 1/00, A61B 1/06

(54) **ENDOSCOPE**

(30) Priority: 16.05.2022 JP 2022080343
(71) Applicant: HOYA CORPORATION, Shinjuku-ku Tokyo 160-8347 (JP)
(72) Inventor: WATANABE, Toshiki, Tokyo 160-8347 (JP)
(74) Representative: Schaumburg und Partner Patentanwälte mbB
(86) International application number: PCT/JP2023/015766
(87) International publication number: WO 2023/223764

(57) **Abstract**

Provided is an endoscope in which unevenness of illumination light is not likely to occur.

An endoscope includes: a translucent tubular distal end cover disposed at a distal end of an insertion portion; a tubular fixation frame in which the distal end cover is fixed on a distal end side thereof; and a tubular illumination frame fixed to an inner peripheral surface of the fixation frame and holding a light source at a distal end thereof, in which a predetermined gap is provided between an inner surface of the distal end cover and surfaces of the illumination frame and the light source. The endoscope further includes an observation window protruding from the distal end cover, in which the distal end cover includes an illumination lens unit that transmits illumination light emitted from the light source and illuminates a visual field direction of the observation window.

## Description

### Technical Field

The present invention relates to an endoscope.

### Background Art

An endoscope having a light-emitting element for illumination at the distal end has been proposed (Patent Literature 1).

### Citation List

### Patent Literature

Patent Literature 1: JP 10-216085 A

### Summary of Invention

### Technical Problem

In order to prevent overlooking of a lesion in an endoscopic examination, it is desirable to use an observation optical system having a wide viewing angle capable of observing a wide range. Since the inside of the gastrointestinal tract is dark, the endoscope needs to have a function of illuminating the observation field of view with illumination light. In the endoscope according to Patent Literature 1, illumination light emitted from a light-emitting element disposed at a distal end illuminates an observation field of view.

However, when there is unevenness in the light distribution of the illumination light, unevenness in brightness due to the illumination light also occurs in the endoscope image. The unevenness in brightness is likely to cause confusion with irregularities or the like of a stomach wall or the like during endoscopic observation, and thus puts a burden on a medical doctor who performs an endoscopic examination.

According to one aspect, an object is to provide an endoscope in which unevenness of illumination light hardly occurs.

### Solution to Problem

An endoscope includes: a translucent tubular distal end cover disposed at a distal end of an insertion portion; a tubular fixation frame in which the distal end cover is fixed on a distal end side thereof; and a tubular illumination frame fixed to an inner peripheral surface of the fixation frame and holding a light source at a distal end thereof, in which a predetermined gap is provided between an inner surface of the distal end cover and surfaces of the illumination frame and the light source.

### Advantageous Effects of Invention

In one aspect, it is possible to provide an endoscope in which unevenness of illumination light hardly occurs.

### Brief Description of Drawings

Fig. 1 is an exterior view of an endoscope.
Fig. 2 is an enlarged perspective view of a distal end portion.
Fig. 3 is a view taken in the direction of arrow III in Fig. 1.
Fig. 4 is a view taken in the direction of arrow IV in Fig. 3.
Fig. 5 is a partial cross-sectional view of a distal end portion taken along line V-V in Fig. 3.
Fig. 6 is a partial cross-sectional view of a distal end portion taken along line VI-VI in Fig. 3.
Fig. 7 is an enlarged view of a region VII in Fig. 6.
Fig. 8 is a partial cross-sectional view of a distal end portion taken along line VIII-VIII in Fig. 5.
Fig. 9 is a partial cross-sectional view of a distal end portion taken along line IX-IX in Fig. 5.
Fig. 10 is a rear view of a distal end cover.
Fig. 11 is a cross-sectional view of the distal end cover taken along line XI-XI in Fig. 10.
Fig. 12 is a perspective view of the distal end cover as viewed from the rear side.
Fig. 13 is a graph illustrating a spectrum of light emitted from a light source.

### Description of Embodiments

### [First Embodiment]

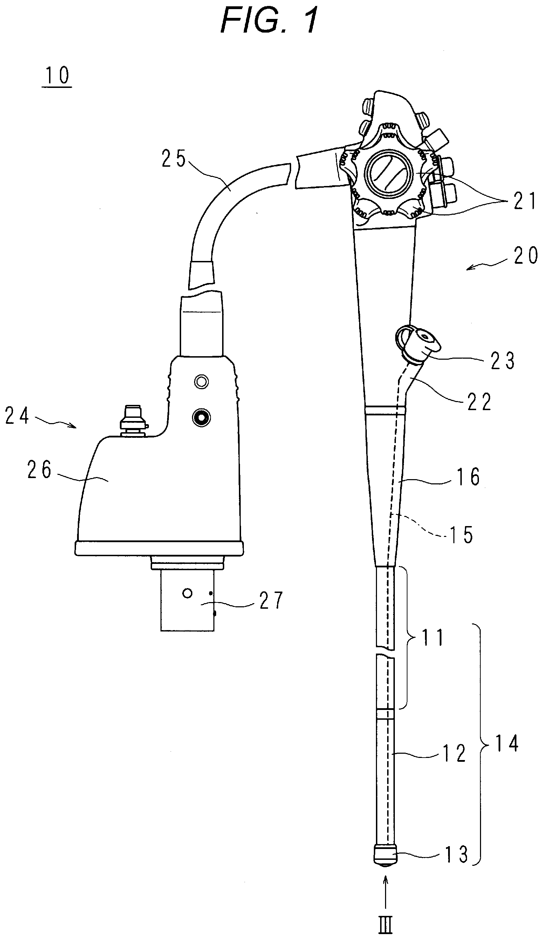

Fig. 1 is an exterior view of an endoscope 10. The endoscope 10 of the present embodiment is a flexible scope for a gastrointestinal tract. The endoscope 10 includes an insertion portion 14, an operation unit 20, a universal cord 25, and a connector unit 24. The operation unit 20 includes a bending knob 21 and a channel inlet 22.

The insertion portion 14 is long and has one end connected to the operation unit 20 via a bend preventing portion 16. The insertion portion 14 includes the soft portion 11, a bending section 12, and a distal end portion 13 in this order from the operation unit 20 side. The bending section 12 is bent according to an operation of the bending knob 21.

A channel 15 penetrating the insertion portion 14 is provided from the channel inlet 22 to the distal end portion 13. A biopsy valve 23 having an insertion port for inserting a treatment tool or the like is attached to the channel inlet 22.

In the following description, a longitudinal direction of the insertion portion 14 is referred to as an insertion direction. Similarly, a side close to the operation unit 20 along the insertion direction is referred to as an operation unit side, and a side distant from the operation unit 20 is referred to as a distal end side.

The universal cord 25 is long, and has a first end connected to the operation unit 20 and a second end connected to the connector unit 24. The connector unit 24 is covered with a substantially rectangular parallelepiped connector case 26. A scope connector 27 protrudes from one surface of the connector case 26. The connector unit 24 is connected to a processor for an endoscope and the like (not illustrated).

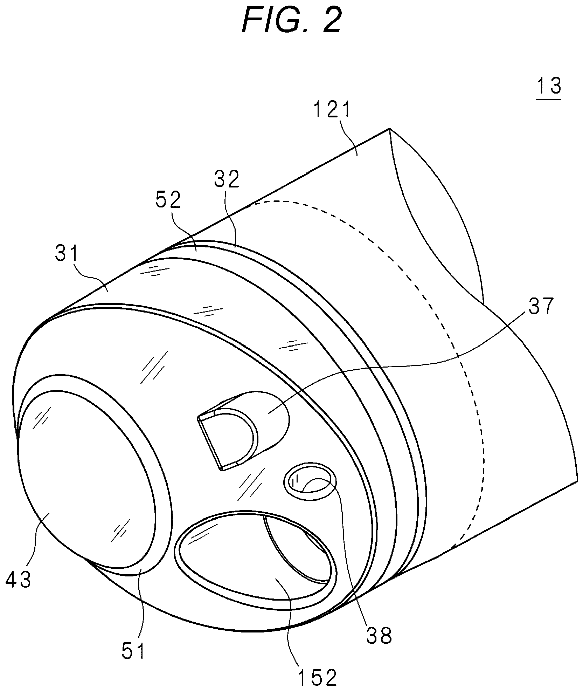

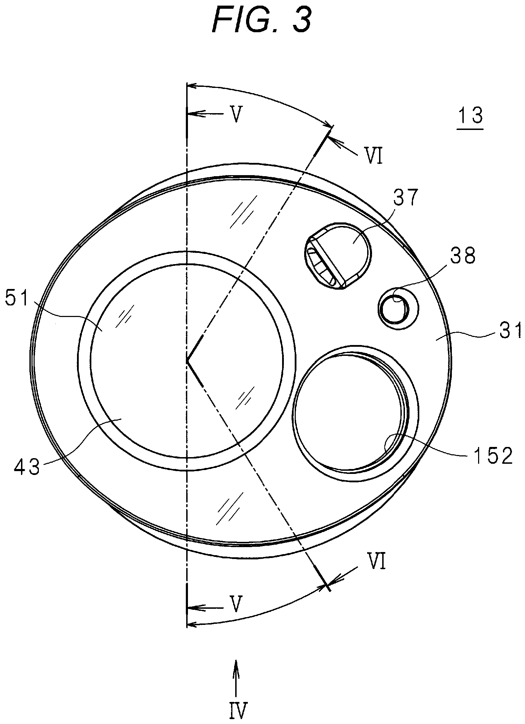

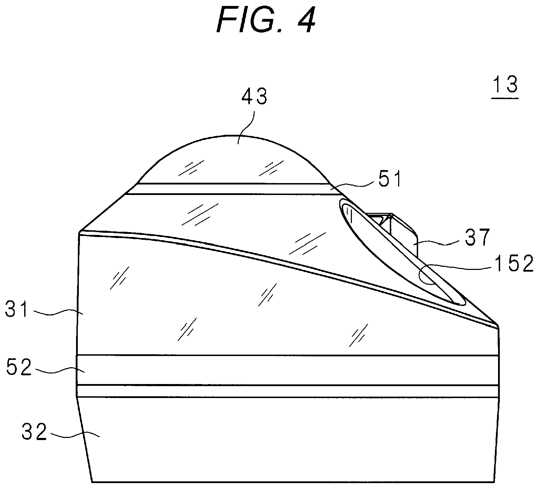

Fig. 2 is an enlarged perspective view of a distal end portion 13. Fig. 3 is a view taken in the direction of arrow III in Fig. 1. Fig. 4 is a view taken in the direction of arrow IV in Fig. 3.

The distal end portion 13 includes a fixation frame 32 and a distal end cover 31 disposed on a distal end side of the fixation frame 32. The distal end cover 31 is translucent. The material of the distal end cover 31 will be described later. The operation unit side of the fixation frame 32 is covered with a bending rubber 121 that is an exterior member of the bending section 12. Components on the operation unit side from the bending section 12, including the bending rubber 121, are not illustrated in the drawings of Fig. 3 and later.

The end surface of the distal end cover 31 has a substantially conical shape. An observation window 43 is disposed at the apex of the substantially conical shape. The observation window 43 is a lens located closest to the object among the lens group constituting the observation optical system. Water tightness is ensured between the edge of the observation window 43 and the distal end cover 31 by the adhesive 51 formed and cured in a gentle shape.

An air/water supply nozzle 37 having a discharge port for discharging air and water toward the observation window 43 is disposed on an end surface of the distal end cover 31. The air/water supply nozzle 37 is used for cleaning the observation window 43 during use of the endoscope 10.

A channel outlet 152 and a distal end water supply hole 38 are opened on an end surface of the distal end cover 31. The channel outlet 152 is an end portion on the distal end side of the channel 15. The treatment tool inserted from the biopsy valve 23 protrudes from the channel outlet 152 via the channel 15.

As illustrated in Fig. 3, the bottom surface of the distal end cover 31 has a substantially elliptical shape having a major axis in the left-right direction in Fig. **3****.** As illustrated in Figs. 3 and 4, the center of the observation window 43 is disposed at a position close to the left side of the distal end cover 31. The air/water supply nozzle 37, the distal end water supply hole 38, and the channel outlet 152 are disposed at positions close to the right side of the distal end cover 31.

Note that Figs. 2 to 4 are examples of an appearance of the end surface of the distal end portion 13, and an arrangement of each member is not limited to Fig. **2****.** For example, instead of the air/water supply nozzle 37, an independent air supply nozzle and water supply nozzle may be provided.

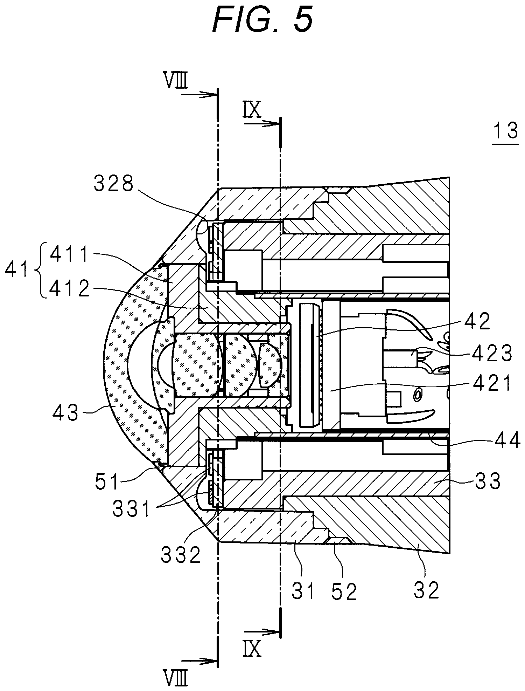

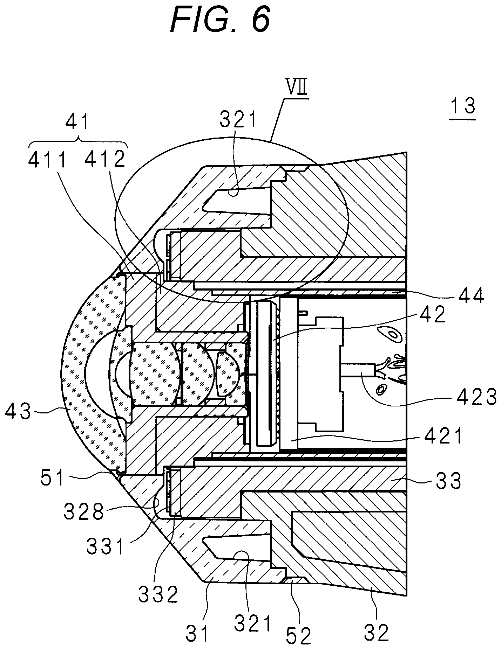

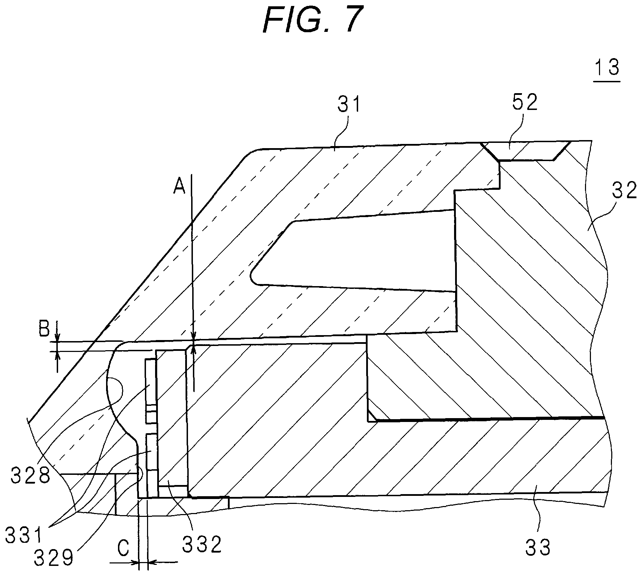

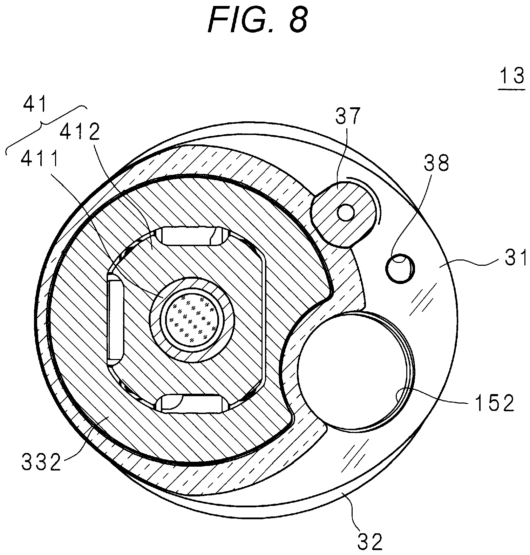

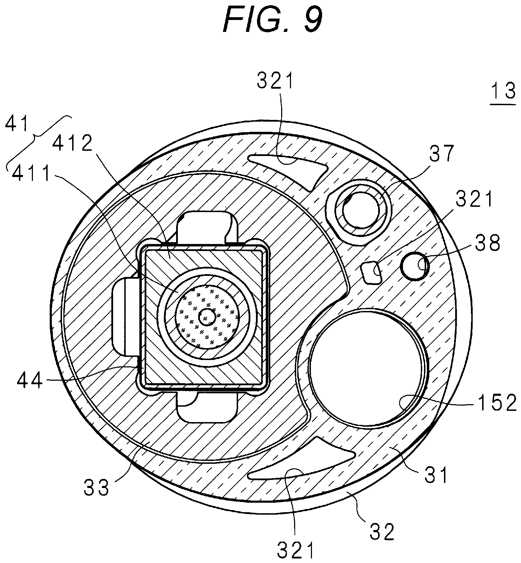

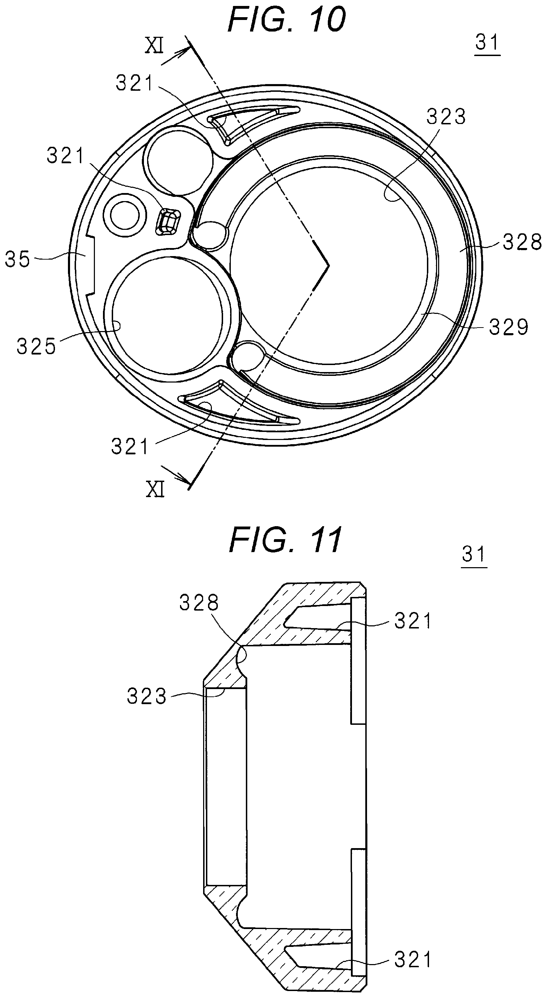

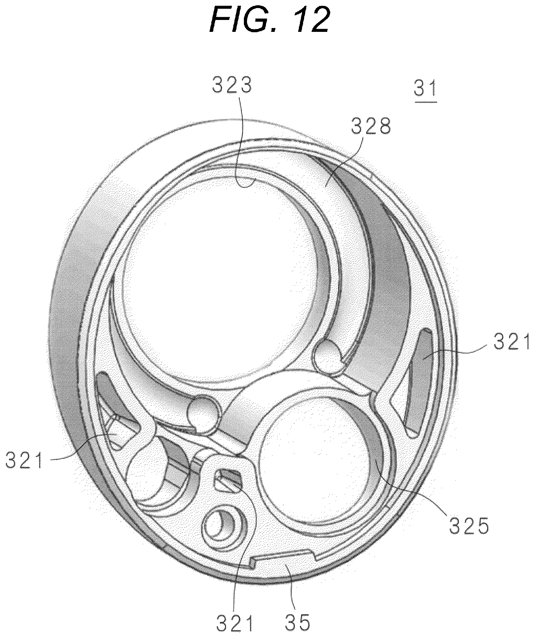

Fig. 5 is a partial cross-sectional view of the distal end portion 13 taken along line V-V in Fig. 3. Fig. 6 is a partial cross-sectional view of the distal end portion 13 taken along line VI-VI in Fig. 3. Fig. 7 is an enlarged view of a region VII in Fig. 6. Fig. 8 is a partial cross-sectional view of a distal end portion 13 taken along line VIII-VIII in Fig. 5. Fig. 9 is a partial cross-sectional view of a distal end portion 13 taken along line IX-IX in Fig. 5. Fig. 10 is a rear view of the distal end cover 31. Fig. 11 is a cross-sectional view of the distal end cover 31 taken along line XI-XI in Fig. 10. Fig. 12 is a perspective view of the distal end cover 31 as viewed from the rear side.

The fixation frame 32 has a substantially cylindrical shape. On the distal end side of the fixation frame 32, an engagement surface tapered stepwise is formed circumferentially. The operation unit side of the distal end cover 31 has a substantially cylindrical shape having, on the inner surface, a stepwise engagement surface to be engaged with the engagement surface of the fixation frame 32. The distal end side of the distal end cover 31 protrudes inward, and the end surface on the distal end side is a substantially conical surface as described above. As illustrated in Fig. 10, the distal end cover 31 has an observation hole 323 on the inner surface of the protruding portion.

The fixation frame 32 and the distal end cover 31 abut against each other over the entire circumference at the stepwise engaging surfaces, and are water-tightly bonded by the adhesive 52. On the outer peripheral portion of the bonded surface, an adhesive reservoir is formed by the adhesive 52 over the entire circumference, and the outer peripheral surface of the fixation frame 32 and the outer peripheral surface of the distal end cover 31 are smoothly connected.

Note that the outer peripheral surface of the fixation frame 32 and the outer peripheral surface of the distal end cover 31 may be smoothly connected to each other by a high-viscosity adhesive after the fixation frame 32 and the distal end cover 31 are bonded and fixed to each other by using a low-viscosity adhesive.

As illustrated in Figs. 5 and 6, a plurality of lenses are disposed on the operation unit side of the observation window 43 described above to constitute an objective optical system together with the observation window 43. Each lens is fixed to a first lens frame 411 having a mirror frame function. A tubular second lens frame 412 is disposed on the operation unit side of the first lens frame 411. A male thread is formed on an outer peripheral surface of the first lens frame 411, and a female thread is formed on an inner peripheral surface of the second lens frame 412. The first lens frame 411 and the second lens frame 412 are coupled by screws to form the lens frame 41.

The image sensor 42 is disposed on the operation unit side of the objective optical system. An optical element such as a microlens array and a cover glass is arranged on the surface of the image sensor 42. The image sensor 42 is mounted on the image sensor substrate 421, and is connected to the image sensor cable 423 via the image sensor substrate 421. A side surface of the image sensor 42 and a connection portion of the image sensor cable 423 are covered with a shield tube 44 that functions as an electromagnetic noise shield. The shield tube 44 is fixed to an outer peripheral surface of the second lens frame 412.

The lens frame 41 is fixed with an adhesive in a state where a fitting length between the male thread of the first lens frame 411 and the female thread of the second lens frame 412 is adjusted such that the focal point of the objective optical system is aligned with the image sensor 42. The outer periphery on the distal end side of the first lens frame 411 and the observation hole 323 of the distal end cover 31 are water-tightly fixed by an adhesive. The first lens frame 411 and the observation hole 323 may also be fixed by the adhesive 51 that fixes the edge of the observation window 43 and the distal end cover 31.

The first lens frame 411, the second lens frame 412, and the shield tube 44 have a light shielding property. Therefore, optical artifacts caused by light having not passed through the objective optical system and being incident on the image sensor 42 are prevented.

A tubular illumination frame 33 is disposed inside the distal end cover 31 and the fixation frame 32. A light source substrate 332 is held on a distal end surface of the illumination frame 33. As illustrated in a cross-sectional view in Fig. 8, the light source substrate 332 has an annular shape. The light source substrate 332 has a concave portion that avoids interference with the channel outlet 152 at one location on the outer periphery.

A plurality of light sources 331 are annularly mounted on the light source substrate 332. The light source 331 has a configuration in which a semiconductor light-emitting element such as a light emitting diode (LED) is covered with a phosphor. The semiconductor light-emitting element is annularly mounted on the light source substrate 332, and is connected to an illumination cable (not illustrated) via the light source substrate 332.

The semiconductor light-emitting element emits light in a narrow band. The phosphor is excited by light emitted from the semiconductor light-emitting element, and emits light in a broader band as compared with the semiconductor light-emitting element. Therefore, the spectrum of the light emitted from the light source 331 does not match the spectrum of the light emitted from the semiconductor light-emitting element.

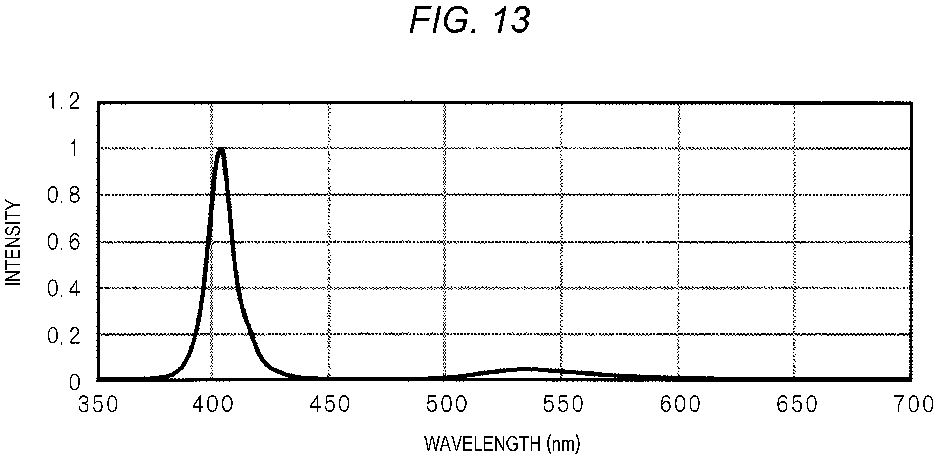

Fig. 13 is a graph illustrating a spectrum of light emitted from the light source 331. In the present embodiment, the semiconductor light-emitting element emits violet light of 405 nanometers, that is, a single wavelength. The phosphor covering the semiconductor light-emitting element is a β-sialon phosphor. The horizontal axis in Fig. 13 represents the wavelength of light, and the unit is nanometer. In Fig. 13, the vertical axis represents the intensity of light. The vertical axis in Fig. 13 is dimensionless so that the maximum value is 1.

As illustrated in Fig. 13, the light source 331 strongly emits light having a wavelength of about 400 to 420 nanometers, that is, violet light. The light source 331 also emits green light having a wavelength of about 530 nanometers. The illumination light having a wavelength of about 405 nanometers emphasizes blood vessels near the mucosal surface. The illumination light having a wavelength of about 530 nanometers emphasizes blood vessels inside the mucous membrane. Therefore, the light source 331 having the spectral characteristics illustrated in Fig. 13 is suitable for so-called special light observation.

Note that, although not illustrated, a white light source used for white light observation is also mounted on the light source substrate 332. A medical doctor operates a switch provided in the operation unit 20 to perform the endoscopic examination while appropriately switching between the special light observation and the white light observation.

As illustrated in Figs. 5 to 7, the distal end cover 31 has an illumination recess 328 on a surface facing the light source 331. As illustrated in Figs. 10 and 12, the illumination recess 328 is a C-shaped groove surrounding the observation hole 323. The bottom surface of the illumination recess 328 has a substantially cylindrical shape.

As illustrated in Figs. 7 and 10, the distal end cover 31 has an annular planar portion 329 on the inner periphery of the illumination recess 328. The planar portion 329 has a planar portion parallel to the distal end surface of the illumination frame 33. As illustrated in Fig. 7, planar portion 329 is disposed parallel to the distal end surface of the illumination frame 33.

The illumination light emitted from the light source 331 annularly arranged around the observation optical system is diffused by the illumination recess 328 having a concave lens shape, and substantially uniformly illuminates the field of view of the image sensor 42. Therefore, it is possible to provide the endoscope 10 in which the light distribution unevenness of the illumination light is small. The illumination recess 328 transmits the illumination light emitted from the light source 331 and realizes a function of an illumination lens unit that illuminates the visual field direction of the observation window 43.

As illustrated in Figs. 9 to 12, the distal end cover 31 has three absorption portions 321 opened to the operation unit side. Each of the absorption portions 321 is a recess that does not penetrate the distal end cover 31. The openings of the two absorption portions 321 are substantially triangular, and the opening of one absorption portion 321 is substantially rectangular. As illustrated in Fig. 11, the bottom portion of the absorption portion 321 is substantially parallel to the end surface on the distal end side of the distal end cover 31.

As illustrated in Figs. 10 and 12, a plate-shaped cover claw 35 protruding inward is provided at an end portion of the distal end cover 31 on the operation unit side. The cover claw 35 is engaged with a recess (not illustrated) provided on a side surface of the fixation frame 32. Therefore, the distal end cover 31 is attached to the fixation frame 32 to a predetermined orientation.

As illustrated in Figs. 7 and 9, a gap is provided over the entire circumference between the outer peripheral surface of the large-diameter portion provided on the distal end side of the illumination frame 33 and the inner surface of the distal end cover 31. Since the distal end cover 31 is manufactured by, for example, injection molding, a draft taper is provided on the inner surface. Due to the influence of the draft taper, the gap between the illumination frame 33 and the distal end cover 31 becomes narrower toward the distal end side. In Fig. 7, the gap between the outer peripheral surface of the illumination frame 33 and the inner surface of the distal end cover 31 at the closest position is indicated by **A.**

As illustrated in Figs. 7 and 8, a gap is also provided over the entire circumference between the outer peripheral surface of the light source substrate 332 and the inner surface of the distal end cover 31. In Fig. 7, the gap between the outer peripheral surface of the light source substrate 332 and the illumination frame 33 and the inner surface of the distal end cover 31 at the closest position is indicated by B. A relationship between the gap A and the gap B is preferably A ≤ B.

As illustrated in Fig. 7, a gap is also provided between the surface on the distal end side of the light source 331 and the planar portion 329. The gap therebetween is indicated by C. Note that, in Fig. 7, the surface on the distal end side of the light source 331 indicates the surface of the phosphor covering the semiconductor light-emitting element, but the definition of the surface on the distal end side is not limited thereto.

For example, when the phosphor and the semiconductor element are enclosed in a case made of a transparent resin, the surface on the distal end side of the light source 331 indicates the surface of the case. When the semiconductor light-emitting element is not covered with the phosphor, the surface on the distal end side of the light source 331 exposes the surface of the semiconductor light-emitting element.

It is desirable that the gap A, the gap B, and the gap C have as small dimensions as possible from the viewpoint of reducing the pain of the patient who receives the endoscopic examination by narrowing the diameter of the distal end portion 13 and shortening the hard portion of the distal end of the endoscope 10. It is not desirable to make the gap A, the gap B, and the gap C zero. The reason why the gaps are necessary will be described later.

The gap A is desirably 50 micrometers or more and 250 micrometers or less. The gap A is more desirably 50 micrometers or more and 140 micrometers or less.

The gap B is desirably 50 micrometers or more and 250 micrometers or less. The gap B is more desirably 90 micrometers or more and 140 micrometers or less.

The gap C is desirably 50 micrometers or more and 250 micrometers or less. The gap C is more desirably 50 micrometers or more and 140 micrometers or less.

The semiconductor light-emitting element converts a part of the supplied electric energy into heat. Therefore, the light source 331 generates heat during use of the endoscope 10. The heat generated from the light source 331 is transferred to the light source substrate 332 and the illumination frame 33 by thermal conduction, and is transferred to the distal end cover 31 by thermal radiation.

As illustrated in Figs. 10 to 12, since the distal end cover 31 has a relatively complex shape, it is desirable to manufacture the distal end cover by injection molding from the viewpoint of manufacturing cost. Therefore, the distal end cover 31 is desirably made of a resin material that can be injection molded.

A material of the distal end cover 31 will be described. As described above, the distal end cover 31 needs to have translucency for transmitting the illumination light emitted from the light source 331. Specifically, in the graph illustrated in Fig. 13, it is desirable that the distal end cover 31 sufficiently transmits light in a range from 380 nanometers where the intensity of the illumination light is high to about 800 nanometers which is the upper limit of visible light.

The light transmittance of the resin material can be measured in accordance with JIS (Japanese Industrial Standards) R 3106:2019 "Testing method for transmittance, reflectance and emissivity of flat glass and calculation of total solar energy transmittance of glazing". For example, when a flat plate-shaped test piece having a thickness of 4 mm is used, the distal end cover 31 is desirably made of a material having a light transmittance of 70% or more, more desirably 80% or more of light having a wavelength in the above range.

As illustrated in Fig. 13, the illumination light emitted from the light source 331 includes ultraviolet rays in a UV (Ultraviolet)-A range having a wavelength of 315-400 nanometers. It is known that a resin material having low ultraviolet resistance causes deterioration such as discoloration and embrittlement by ultraviolet irradiation. In a case where the distal end cover 31 is discolored, the illumination light emitted from the light source 331 is uneven. In a case where the distal end cover 31 becomes brittle, there is a possibility that the function and safety of the endoscope 10 are impaired, for example, a crack occurs during endoscopic examination or reprocessing. Therefore, the distal end cover 31 needs to have ultraviolet resistance.

The distal end cover 31 is disposed at the distal end of the insertion portion 14. Therefore, it is necessary to have strength enough not to be damaged even when being pressed against a stomach wall or the like during an endoscopic examination. When abrasion occurs on the surface of the distal end cover 31 due to mechanical abrasion such as brushing performed at the time of reprocessing of the endoscope 10, the translucency of the illumination light is deteriorated. Therefore, the distal end cover 31 is also required to have such hardness that abrasion does not occur by brushing or the like. The distal end cover 31 also needs to have resistance to various drugs used for reprocessing of the endoscope 10.

Further, the distal end cover 31 is water-tightly bonded to the fixation frame 32, the first lens frame 411, and the observation window 43 as described above. That is, the distal end cover 31 needs to be made of an adhesive material.

Note that the various conditions described above may be realized by a combination of the material constituting the distal end cover 31 and the coating applied to the surface of the distal end cover 31.

According to the investigation by the applicant, materials shown in Table 1 may satisfy various conditions as exemplified above.

**[Table 1]**

| Material Name |
|---|
| Polyamide |
| Polycarbonate |
| Copolyesther |
| Cyclo-Olefin Polymer |

For each resin shown in Table 1, various products are provided from resin manufacturers. For example, regarding polyamide, products shown in Table 2 are promising as a material of the distal end cover 31.

**[Table 2]**

| Manufacturer | Product Name |
|---|---|
| EMS-CHEMIE (Japan) Ltd. | Grilamid (Registered Trademark) |
| Arkema (Registered Trademark) | Rilsan (Registered Trademark) |
| Polyplastics-Evonik Corporation | TROGAMID (Registered Trademark) |
| UNITIKA Ltd. | UNITIKA Nylon 6 |

Grilamid is an example of polyamide 12, which is an aliphatic polyamide. Rilsan is an example of polyamide 11 which is an aliphatic polyamide. TROGAMID is an example of amorphous polyamides and microcrystalline polyamides. UNITIKA nylon 6 is an example of a semi-crystalline polyamide.

Resins of various grades are further supplied from resin manufacturers for each product. Whether or not the material of the distal end cover 31 can be adopted needs to be determined by performing a test for each grade.

In general, it is known that a resin material has a higher water absorption rate and a higher linear expansion coefficient than materials such as stainless steel and glass. Further, the materials illustrated in Tables 1 and 2 have a higher water absorption rate and a higher linear expansion coefficient than engineering plastics that have been conventionally used for the endoscope 10. Examples of the water absorption rate and the linear expansion coefficient for each material are shown in Table 3.

In Table 3, Noryl with glass filler is an example of an engineering plastic that has been conventionally used for the endoscope 10. Polyamide is an example of the resin material shown in Table 1. The properties of the material vary depending on the grade. Table 3 shows an example of measurement using a representative grade appropriately selected from each material.

Water absorption rate is a value measured according to ASTM D570 (Standard Test Method for Water Absorption of Plastics: Standard Test Method for Water Absorption of Plastics). The linear expansion coefficient is a value measured according to ASTM D696 (Standard Test Method for Coefficient of Linear Thermal Expansion of Plastics Between -30°C and 30°C with a Vitreous Silica Dilatometer).

**[Table 3]**

| Material | Water Absorption Rate [%/24h] | Linear Expansion Coefficient [ppm/°C] |
|---|---|---|
| Glass | 0 | 14 |
| Stainless Steel | 0 | 7 |
| Noryl With Glass Filler | 0.06 | 52 |
| Polyamide | 0.4 | 70 |

As shown in Table 3, Noryl with glass filler, which is a resin material, has a higher water absorption rate and a higher linear expansion coefficient than glass and stainless steel. The water absorption rate and the linear expansion coefficient of the polyamide are larger than the water absorption rate and the linear expansion coefficient of Noryl with glass filler. Although data is omitted, each of the resins shown in Tables 1 and 2 exerts a higher water absorption rate and a higher linear expansion coefficient than those of Noryl with glass filler.

The description will be continued returning to Figs. 6 and 7. The fixation frame 32 and the illumination frame 33 are made of a material conventionally used for the endoscope 10, such as Noryl with glass filler. The light source substrate 332 is, for example, a glass epoxy substrate or a ceramic substrate. The distal end cover 31 is made of a material exemplified in Tables 1 and 2, such as polyamide.

Even when the distal end cover 31 absorbs water and thermally expands, most of the deformation amount of the distal end cover 31 is absorbed by the absorption portion 321. Due to the presence of the gap A, the gap B, and the gap C, the distal end cover 31 does not come into contact with the illumination frame 33 or the light source substrate 332 even when water absorption and thermal expansion occur in the distal end cover 31 and thermal expansion occurs in the illumination frame 33 and the light source substrate 332. Furthermore, detachment of the adhesive 51 and the adhesive 52 due to the difference in thermal expansion coefficient can also be avoided.

Therefore, for example, it is possible to provide the endoscope 10 that is not damaged by water absorption and thermal expansion even in a case where the endoscope 10 reprocessed in a state of being immersed in a chemical solution and hot water is immediately used for endoscopic examination and the light source 331 generates heat.

According to the present embodiment, the material of the distal end cover 31 can be selected with priority given to characteristics such as translucency. Therefore, it is possible to provide the endoscope 10 in which the unevenness of the illumination light hardly occurs.

Note that the endoscope 10 is not limited to a flexible endoscope for the gastrointestinal tract. The endoscope 10 may also be, for example, a respiratory endoscope, a urinary endoscope, or the like. The endoscope 10 may also be a rigid endoscope. The endoscope 10 may also be an industrial endoscope used for inspection of pipes and the like. The visual field direction of the observation window 43 is not limited to the front of the insertion portion 14. That is, the endoscope 10 may be of the side-viewing shape, the anterior oblique-viewing shape, or the backward oblique-viewing shape.

The technical features (constituent elements) described in the embodiments can be combined with each other to form novel technical features in combination.

It should be noted that the embodiments disclosed herein are illustrative in all respects and are not restrictive. The scope of the present invention is defined not by the foregoing meanings but by the claims and is intended to include meanings equivalent to the claims and all modifications within the scope.

### Reference Signs List

- 10: Endoscope
- 11: Soft portion
- 12: Bending section
- 121: Bending rubber
- 13: Distal end portion
- 14: Insertion portion
- 15: Channel
- 152: Channel outlet
- 16: Bend preventing portion
- 20: Operation unit
- 21: Bending knob
- 22: Channel inlet
- 23: Biopsy valve
- 24: Connector unit
- 25: Universal cord
- 26: Connector case
- 27: Scope connector
- 31: Distal end cover
- 32: Fixation frame
- 321: Absorption portion
- 323: Observation hole
- 328: Illumination recess (illumination lens unit)
- 329: Planar portion
- 33: Illumination frame
- 331: Light source
- 332: Light source substrate
- 35: Cover claw
- 37: Air/water supply nozzle
- 38: Distal end water supply hole
- 41: Lens frame
- 411: First lens frame
- 412: Second lens frame
- 42: Image sensor
- 421: Image sensor substrate
- 423: Image sensor cable
- 43: Observation window
- 44: Shield tube
- 51: Adhesive
- 52: Adhesive

## Claims

1. An endoscope comprising:
a translucent tubular distal end cover disposed at a distal end of an insertion portion;
a tubular fixation frame in which the distal end cover is fixed on a distal end side thereof; and
a tubular illumination frame fixed to an inner peripheral surface of the fixation frame and holding a light source at a distal end thereof, wherein
a predetermined gap is provided between an inner surface of the distal end cover and surfaces of the illumination frame and the light source.

2. The endoscope according to claim 1, further comprising
an observation window protruding from the distal end cover, wherein
the distal end cover includes an illumination lens unit that transmits illumination light emitted from the light source and illuminates a visual field direction of the observation window.

3. The endoscope according to claim 2, wherein
the distal end cover includes a planar portion between the illumination lens unit and the observation window, and
a gap between the planar portion and a surface of the light source is 50 micrometers or greater and 250 micrometers or less.

4. The endoscope according to claim 1, wherein
a gap between the inner peripheral surface of the distal end cover and an outer peripheral surface of the illumination frame is 50 micrometers or greater and 250 micrometers or less.

5. The endoscope according to claim 1, wherein
the light source includes a light-emitting element and a phosphor covering the light-emitting element, and
a light source substrate on which the light-emitting element is mounted is held on a distal end surface of the illumination frame.

6. The endoscope according to claim 5, wherein
the light source substrate is in an annular shape, and
a gap between the inner peripheral surface of the distal end cover and an outer peripheral surface of the light source substrate is 50 micrometers or greater and 250 micrometers or less.

7. The endoscope according to claim 5, wherein
the light-emitting element emits violet light, and
the phosphor is excited by light emitted from the light-emitting element and emits green light.

8. The endoscope according to claim 1, wherein
the distal end cover is made of a material having a higher water absorption rate than a material constituting the fixation frame.

9. The endoscope according to claim 1, wherein
the distal end cover is made of a material having a higher linear expansion coefficient than a material constituting the fixation frame.

10. The endoscope according to claim 1, wherein
the distal end cover is made of polyamide, polycarbonate, copolyester, or a cyclo-olefin polymer.

11. The endoscope according to any one of claims 1 to 9, wherein
the distal end cover is made of Grilamid (registered trademark), Rilsan (registered trademark), TROGAMID (registered trademark), or UNITIKA Nylon 6.
